# EUROPEAN PATENT APPLICATION

(11) **EP 2 816 043 A1**
(43) Date of publication of application: **24.12.2014**
(21) Application number: 13173184.6
(22) Date of filing: 21.06.2013
(51) Int. Cl.: C07D 487/04, A61K 9/20, A61K 31/519

(54) **Spherical ticagrelor particles**

(71) Applicant: LEK Pharmaceuticals d.d., 1526 Ljubljana (SI)
(72) Inventor: The designation of the inventor has not yet been filed
(74) Representative: Kunic Tesovic, Barbara

(57) **Abstract**

The present invention relates to crystalline ticagrelor in form of spherical particles, to processes of preparing the same and to the use of the spherical particles for the preparation of a pharmaceutical composition.

## Description

The present invention relates to crystalline ticagrelor in form of spherical particles, to processes of preparing the same and to the use of the spherical particles for the preparation of a pharmaceutical composition.

### Background of the invention

Ticagrelor (TCG; 3-[7-[[(1*R*,2*S*)-2-(3,4-difluorophenyl)cyclopropyl]amino]-5-(propylthio)-3*H*-*1,2*, 3-triazolo[4,5-*d*]pyrimidin-3-yl]-5-(2-hydroxyethoxy)-(1*S*,2*S*,3*R*,5*S*)-1,2-cyclopentanediol) having the following structural formula 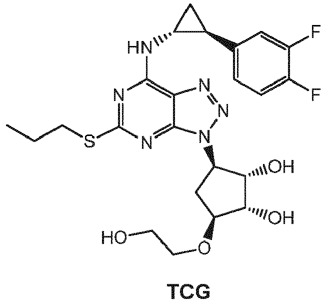 shows pharmaceutical activity by functioning as a P2Y12 receptor antagonist and thus is indicated for the treatment or prevention of thrombotic events, for example stroke, heart attack, acute coronary syndrome or myocardial infection with ST elevation, other coronary artery diseases and arterial thrombosis as well as other disorders related to platelet aggregation (WO 2000/34283).

WO 2001/92262 discloses crystalline forms I, II, III, IV and amorphous ticagrelor. In addition, processes for the production of crystalline ticagrelor by conventional crystallization processes, such as cooling crystallization, antisolvent crystallization or solvent evaporation are disclosed. The crystals of ticagrelor disclosed in WO 2001/92262 are obtained in form of needles or plates, which are crystal habits known for limited flowability and compressibility.

Due to physical and mechanical properties of crystalline ticagrelor the pharmaceutical compositions comprising ticagrelor have been prepared as described in WO 2008/024045 by a wet granulation process comprising the steps of: (a) blending ticagrelor with one or more diluents, disintegrants and binders; (b) wet granulating the blend with water; (c) drying the granules; (d) milling the granules; (e) blending the milled granules with one or more lubricants; and (f) compressing tablets from the blend.

Direct compression is known to be the most efficient and economic process used in tablet manufacturing due to its high speed, simplicity and low costs. However, good flowability and compressibility of the drug substance are prerequisite in order to prepare tablets by direct compression.

There is a need for a ticagrelor crystal habit with improved physical and mechanical properties that would provide a suitable material for direct compression tableting.

### Summary of the invention

The present invention provides ticagrelor in a form of spherical particles which possess improved physical and mechanical properties compared to crystal habits currently known from the art, allowing for the manufacture of tablets by direct compression.

Furthermore, the present invention provides processes for production of said spherical particles as well as their use for the preparation of pharmaceutical compositions.

### Brief description of the drawings

- FIG. 1:: Crystalline spherical ticagrelor particles prepared according to Example 1 of the present invention.
- FIG. 2:: Electron microscope image of isolated crystalline spherical ticagrelor particles obtained according to Example 1 of the present invention.
- FIG. 3:: Electron microscope image of isolated crystalline spherical ticagrelor particles in polymorphic form II obtained according to Example 2 of the present invention.
- FIG. 4:: Needle shaped ticagrelor crystals prepared according to Reference Example 1 of the present invention.
- FIG. 5:: Electron microscope image of isolated needle shaped ticagrelor crystals obtained according to Reference Example 1 of the present invention.
- FIG. 6:: X-Ray diffraction pattern of crystalline ticagrelor obtained by Example 2.

- FIG. 7:: DSC thermogram of crystalline ticagrelor obtained by Example 2.

### Detailed description of the invention

Aspects, advantageous features and preferred embodiments of the present invention will be described in further detail below, noting however that such aspects, advantageous features as well as embodiments and examples are presented for illustrative purposes only and shall not limit the invention in any way.

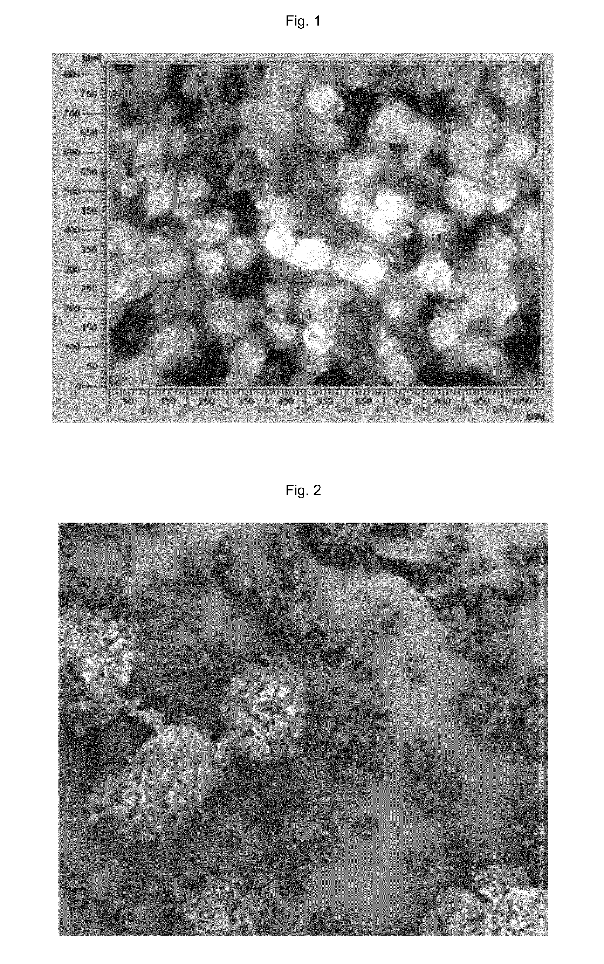

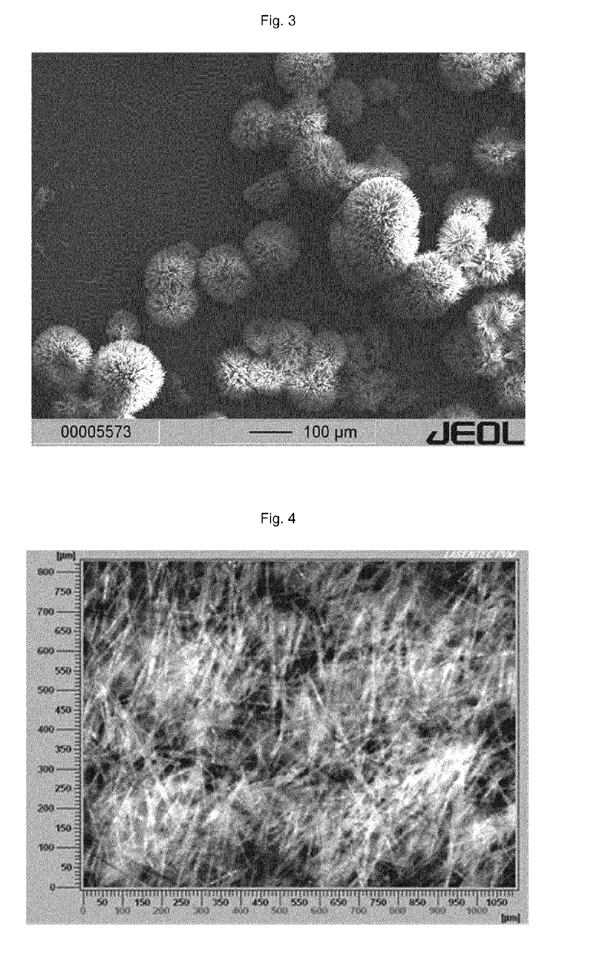

In one embodiment of the disclosure, crystalline ticagrelor in form of spherical particles is provided. The term "spherical particles" as used herein refers to sphere shaped agglomerates. Representative electron microscope images of the crystalline spherical ticagrelor particles of the present embodiment are provided in Figures 2 and 3.

A typical d₉₀ particle size of the crystalline spherical ticagrelor particles of the present invention ranges from about 50 to 1000 µm, preferably from about 75 to 500 µm. In particular, the d₉₀ particle size ranges from about 100 to 350 µm determined by laser diffraction.

The term "about" as used herein means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10 %.

The crystalline spherical ticagrelor particles of the present invention may be present as polymorphically pure form II, polymorphically pure form III or a mixture of polymorphs II and III. The solid state form of the crystalline spherical ticagrelor particles can be determined by well-known methods, such as differential scanning calorimetry (DSC) or X-ray powder diffraction (XRPD). Preferably, a combination of both DSC and XRPD is used in order to unambiguously identify the solid state form of the crystalline spherical ticagrelor particles.

In another embodiment the present invention relates to a process for the preparation of crystalline spherical ticagrelor particles comprising the steps of:
(i) providing a solution of ticagrelor in a solvent,
(ii) adding the obtained solution to an antisolvent,
(iii) isolating the obtained precipitated crystalline spherical ticagrelor particles, and
(iv) drying the isolated crystalline spherical ticagrelor particles.

Any form of ticagrelor can be applied as a starting material in the process e.g. crystalline ticagrelor, amorphous ticagrelor or mixtures thereof. Suitable crystalline forms are e.g. forms I, II, III and IV described in WO 2001/92262. Crude ticagrelor can be prepared e.g. according to the procedure disclosed in the example 3 of WO 2000/34283.

In the first step a solution of ticagrelor is prepared. The solvent is selected from the group consisting of methyl acetate, ethyl acetate, n-propyl acetate, isopropyl acetate, n-butyl acetate, isobutyl acetate, and a mixture thereof. Preferably isopropyl acetate is applied as a solvent.

The initial ticagrelor concentration in the solvent ranges from about 50 to 150 g/L, preferably from about 75 to 125 g/L, and most preferably the concentration applied is about 100 g/L.

The applied ticagrelor starting material is dissolved upon heating. The dissolution temperature depends on the applied solvent and the initial ticagrelor concentration and ranges from about 55°C to 125°C. Preferably, the mixture is heated to the reflux temperature of the applied solvent in order to completely dissolve the starting material.

After the ticagrelor starting material is dissolved, an optional filtration step may be applied in order to remove any possible undissolved particles. Moreover, if there is a need for decolorization, the solution may be treated with charcoal prior to filtration.

Thereafter the hot ticagrelor solution is charged into a crystallizer comprising an antisolvent selected from the group consisting of toluene, n-heptane, isooctane, and a mixture thereof. The preferred antisolvent is toluene.

The solvent : antisolvent ratio ranges from about 1 : 0.5 to 1 : 5 (volume : volume).

The addition rate of charging the ticagrelor solution into a crystallizer comprising the antisolvent is not critical for the crystal habit obtained. Hence, the hot ticagrelor solution can be poured at once into the crystallizer comprising the antisolvent or may be slowly added, for example within a period of about 0.5 to 6 hours.

The temperature in the crystallizer comprising the antisolvent should not exceed about 10°C, more preferably about 5°C and most preferably the temperature in the crystallizer comprising the antisolvent is about 0°C during the precipitation of the crystals. If the temperature in the crystallizer during addition of ticagrelor solution to antisolvent is higher, the formation of needle shaped product is favored.

In the next step, the suspension can optionally be stirred for a period of up to about 2 hours to achieve equilibrium solubility whereat the yield is maximized, and then the obtained crystalline spherical ticagrelor particles are collected by a conventional method such as filtration or centrifugation.

Finally, the isolated crystalline spherical particles are dried by conventional means, for example vacuum drying or fluidized bed drying, at a temperature ranging from about 10°C to 60°C, preferably from about 15°C to 50°C and most preferably from about 25°C to 40°C for a period of time ranging from about 1 to 72 hours, preferably from about 6 to 48 hours and most preferably from about 12 to 24 hours.

In a particularly preferred embodiment the crystalline spherical ticagrelor particles are prepared by the following process: ticagrelor is dissolved in isopropyl acetate upon heating the mixture to the reflux temperature. Next, the hot ticagrelor solution is charged into a crystallizer containing toluene, while keeping the reaction temperature of crystallizer at 0°C during the entire step of addition of the solution to the antisolvent. In the next step the obtained suspension is stirred for two hours at 0°C before the crystalline spherical ticagrelor particles are collected on a filter. Finally, the obtained crystalline spherical ticagrelor particles are vacuum dried at 35°C for 24 hours.

In another embodiment the present invention relates to a process for the preparation of crystalline spherical ticagrelor particles comprising the steps of:
(i) providing a solution of ticagrelor in a solvent,
(ii) adding an antisolvent to the obtained solution,
(iii) isolating the obtained precipitated crystalline spherical ticagrelor particles, and
(iv) drying the isolated crystalline spherical ticagrelor particles.

Any form of ticagrelor can be applied as a starting material in the process e.g. crystalline ticagrelor, amorphous ticagrelor or mixtures thereof. Suitable crystalline forms are e.g. forms I, II, III and IV described in WO 2001/92262. Crude ticagrelor can be prepared e.g. according to the procedure disclosed in the example 3 of WO 2000/34283.

In the first step a solution of ticagrelor is prepared. The solvent is selected from the group consisting of methyl acetate, ethyl acetate, n-propyl acetate, isopropyl acetate, n-butyl acetate, isobutyl acetate, and a mixture thereof. Preferably ethyl acetate is applied as a solvent.

The initial ticagrelor concentration in the solvent ranges from about 50 to 150 g/L, preferably from about 75 to 125 g/L, and most preferably the concentration applied is about 100 g/L.

The applied ticagrelor starting material is dissolved upon heating. The dissolution temperature depends on the applied solvent and the initial ticagrelor concentration and ranges from about 55°C to 125°C. Preferably the mixture is heated to the reflux temperature of the applied solvent in order to completely dissolve the starting material.

After the ticagrelor starting material is dissolved, an optional filtration step may be applied in order to remove any possible undissolved particles. Moreover, if there is a need for decolorization, the solution may be treated with charcoal prior to filtration.

Thereafter the solution is adjusted to a temperature of about 25 - 65°C. Crystallization is initiated by the addition of an antisolvent, whereat the previously adjusted temperature is kept constant during the whole antisolvent addition.

Careful selection of crystallization temperature enables that the obtained crystalline spherical ticagrelor particles consist of a pure specific polymorphic form. In order to obtain spherical ticagrelor with polymorphically pure form II it is essential to adjust and keep the temperature at 45-55 °C during the crystallization. At lower crystallization temperatures during the antisolvent addition, mixtures of polymorph II with polymorph III or pure polymorph III may be obtained, whereas higher crystallization temperatures result in mixtures of form II with form I.

The antisolvent is selected from the group consisting of toluene, n-heptane, isooctane, and a mixture thereof. The preferred antisolvent is n-heptane.

A solvent : antisolvent ratio ranges from about 1 : 1 to 1 : 5 (volume : volume). In case the solvent : antisolvent ratio of 1 : 2 is used, the yield is significantly increased compared to 1 : 1 ratio. At ratios with more antisolvent, e.g. 1 : 3 to 1 : 5, yield is similar to the one obtained at solvent : antisolvent ratio of 1 : 2.

In order to obtain spherical particles the antisolvent should be added within about 60 minutes, preferably within about 30 minutes and more preferably within about 15 minutes from the start of addition of the antisolvent into the ticagrelor solution. If the antisolvent is added in a slower manner, for example after 75 minutes from the start of addition of the antisolvent into the ticagrelor solution, the formation of needle shaped product is favored (see Reference Example 2).

After complete antisolvent addition the obtained suspension is cooled to about 25°C. The cooling step is conducted within 1 hour, preferably within 30 minutes. If cooling times above about 1 hour are applied, a conversion of crystalline polymorph II into the thermodynamically stable polymorph III may occur.

In the next step, the obtained crystalline spherical ticagrelor particles are collected by a conventional method such as filtration or centrifugation. In case crystalline spherical ticagrelor particles consisting of a pure polymorphic form II are obtained it is essential to immediate collect the obtained crystalline spherical ticagrelor particles and avoid longer stirring times, for example longer than 1 hour, in order to prevent the conversion of crystalline polymorph II into the thermodynamically stable polymorph III during agitation.

Finally, the isolated crystalline spherical particles are dried by conventional means, for example vacuum drying or fluidized bed drying, at a temperature ranging from about 10°C to 60°C, preferably from about 15°C to 50°C and most preferably from about 25°C to 40°C for a period of time ranging from about 1 to 72 hours, preferably from about 6 to 48 hours and most preferably from about 12 to 24 hours.

In a particularly preferred embodiment the crystalline spherical ticagrelor particles of a pure polymorphic form II are prepared by the following process: ticagrelor is dissolved in ethyl acetate upon heating the mixture to the reflux temperature. Next, the hot ticagrelor solution is cooled to about 55°C. To the ticagrelor solution n-heptane is added within 10 minutes while keeping the reaction temperature at about 50°C during the entire step of addition of the antisolvent to the solution. In the next step the obtained suspension is cooled to about 25°C before the crystalline spherical ticagrelor particles are collected on a filter. Finally, the obtained crystalline spherical ticagrelor particles of a pure polymorphic form II are vacuum dried at 25°C for 16 hours. Crystalline spherical ticagrelor particles according to this embodiment consist of agglomerated needles radiating from a common center.

In another embodiment, the crystalline spherical ticagrelor particles of the present invention can be used in medicine. They may be used alone or in combination, or formulated with one or more excipients or other active pharmaceutical ingredients to provide formulations suitable for the treatment or prevention of arterial thrombotic complications in patients with coronary artery, cerebrovascular or peripheral vascular disease.

The pharmaceutical composition comprising the crystalline spherical ticagrelor particles of the present invention may suitably be in the form of tablets, pills, capsules, powders or granules for oral administration.

Suitable excipients and/or carriers include, without being limited to, diluents, binders, disintegrants, lubricants, etc. For example, the crystalline spherical ticagrelor particles, are mixed with a carrier or binder substance, e.g. a mono-, di- or polysaccharide such as sugars and starch, a sugar alcohol or another polyol. For example, lactose, saccharose, sorbitol, mannitol, starch, cellulose derivatives, a binder such as polyvinylpyrrolidone, and a lubricant such as magnesium stearate, calcium stearate, polyethylene glycol, waxes, paraffin, and the like are mixed, and then compressed into tablets. The particles of the present invention or the tablets containing the same may be coated by another substance. The powder mixture containing the particles of the present invention may also be dispensed into capsules.

In a preferred aspect of the present invention, a pharmaceutical composition comprising the crystalline spherical ticagrelor particles prepared according to the present invention can be prepared by dry mixing the provided crystalline ticagrelor in form of spherical particles with the pharmaceutically acceptable carrier or diluent, and directly compressing the obtained admixture into a tablet.

Suitable flowability and compressibility of the drug substance constitute a prerequisite for direct compression tableting. The compressibility index reflects the compressibility of a powder and there is a correlation between this index and the flowability of the crystals. A compressibility index greater than 25% is considered to be an indication for poor flowability, whereas a compressibility index below 15% is an indication for good flowability.

In addition the angle of repose also gives a hint about the flow properties of powders. Generally, when the angle of repose exceeds 50°, the powder flow is rarely acceptable for manufacturing purposes.

The crystalline spherical ticagrelor particles prepared according to the present invention possess improved physical and mechanical properties when compared to the known prior art crystal habit, i.e. ticagrelor needles, and therefore render direct compression tableting possible.

In the following the present invention will be described in further detail by illustrative, nonlimiting examples.

### Examples

### Reference Example 1: Preparation of ticagrelor needles via conventional antisolvent crystallization

63.02 g ticagrelor were dissolved in 420 mL ethyl acetate upon heating to reflux. The obtained solution was cooled to 50 °C and 405 mL isooctane were added within 100 minutes, whereat the temperature of the mixture was kept at 50 ± 5 °C during the whole antisolvent addition. The obtained suspension was stirred for an additional hour at 50 °C before cooling it to 0 °C within 140 minutes. After further stirring for 140 minutes at 0 °C the crystals were filtered off and dried under vacuum (≤ 50 mbar) at 35 °C for 24 hours. 54.78 g (87% yield) needle shaped polymorph II of ticagrelor with a particle size of d(90%)=72 µm were obtained.

### Reference Example 2: Preparation of ticagrelor needles via conventional antisolvent crystallization

A mixture of 33.01 g ticagrelor in 300 mL ethyl acetate was heated to reflux by adjusting a jacket temperature of 80 °C, whereat a clear solution was obtained. Then the jacket temperature was cooled to 55 °C within one hour. The crystallization was initiated by adding 600 mL n-heptane within 90 minutes, whereat the mass temperature was kept at 55 ± 2 °C during the complete addition. The thus obtained suspension was cooled to 25 °C within 15 minutes and the solid material was isolated by filtration. Finally the product was dried under vacuum (≤ 100 mbar) at 25 °C overnight to obtain 29.15 g (88.3% of theory) of ticagrelor needles in polymorphically pure form II.

### Example 1: Preparation of spherical ticagrelor particles via inverse crystallization

15 g of ticagrelor (e.g. prepared according to the procedure disclosed in example 3 of WO 2000/34283) were dissolved in 150 mL of isopropyl acetate upon heating to 73°C (dissolution observed at about 66°C). The obtained hot ticagrelor solution was then charged within one hour into a crystallizer containing 400 mL of toluene, whereat the reaction temperature of the crystallizer was kept at 0°C during the whole addition. After complete addition the obtained suspension was further stirred for two hours at 0°C. Thereafter the solid material was collected on a filter and washed with 100 mL of toluene. The filter cake was vacuum dried (15mbar) at 35°C for 24 hours. 11.54 g spherical ticagrelor particles having a particle size of d(0.9)=126 µm were obtained.

### Example 2: Preparation of spherical ticagrelor form II particles

A mixture of 48.6 g ticagrelor (e.g. prepared according to the procedure disclosed in example 3 of WO 2000/34283) in 490 mL ethyl acetate was heated to a reaction temperature of 75°C, whereat a clear solution was obtained. Then the solution was cooled to 54°C within one hour. The crystallization was initiated by the fast addition of 980 mL n-heptane within 12 minutes, whereat the reaction temperature was kept at 50 ± 5°C during the complete antisolvent addition. The thus obtained suspension was cooled to 25°C within 20 minutes and the solid material was collected on a filter. Finally the product was dried under vacuum (≤30 mbar) at 40°C overnight to obtain 46.9 g (97% of theory) of spherical ticagrelor particles in polymorphically pure form II.

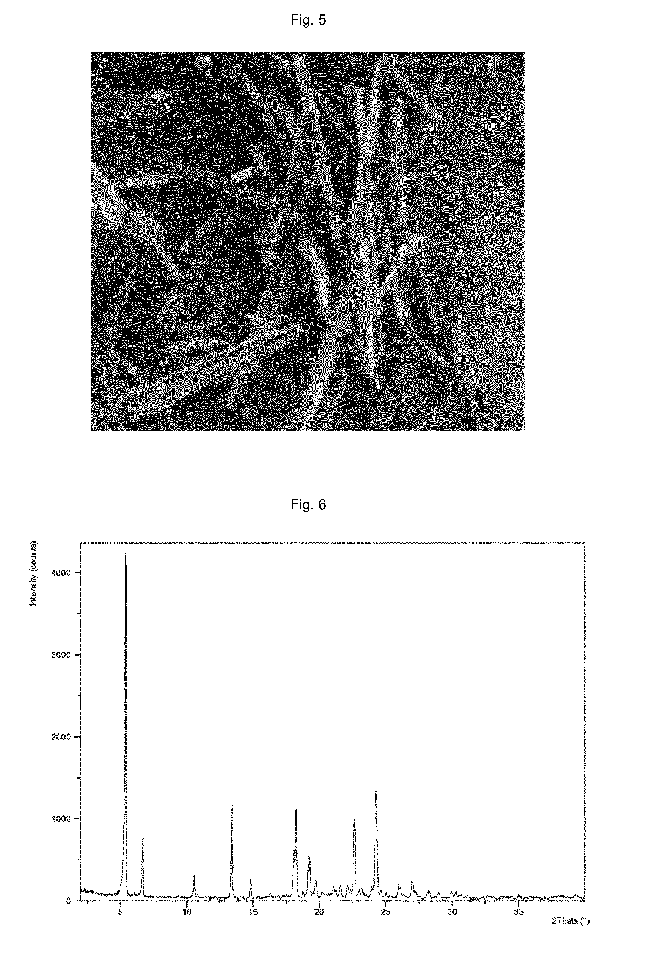

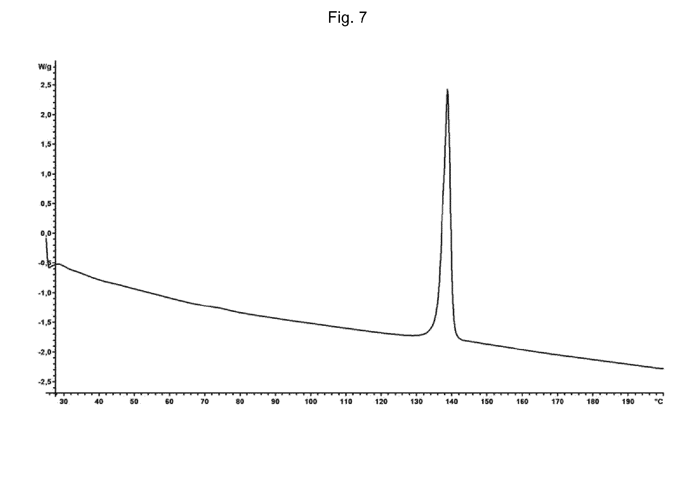

The so obtained polymorph II of ticagrelor was characterized by X-Ray powder diffraction (Table 1, Fig. 6) and differential scanning calorimetry (DSC). A DSC curve presented in Fig. 7 shows a single melting endotherm with an onset temperature of about 136°C and a peak maximum of about 139°C.

**Table 1: Characteristic XRPD angles 2-theta and relative intensities of polymorph II of ticagrelor prepared according to example 2 of the present invention**

| **angle [2-theta]** | **relative intensity [%]** |
|---|---|
| 5.4 | 100 |
| 6.7 | 18 |
| 10.6 | 6 |
| 13.4 | 26 |
| 14.8 | 5 |
| 18.3 | 24 |
| 19.2 | 11 |
| 22.6 | 21 |
| 24.3 | 29 |
| 27.0 | 5 |

### Methods of analysis

### Particle size distribution:

Particle size distribution was determined by laser diffraction method using a Malvern Mastersizer 2000 instrument. A small quantity (-20mg) of the sample was suspended in instrument dispersion cell in hexane together with ~100 mg dioctyl-sulfosuccinate as surfactant. Measurement is performed after 3 min of 30% US. If after 3 min a high right hand peak due to temperature change of hexane refractive index persists, the measurement is repeated after 1 min.

Instrument settings were as follows: Dispersion cell: Hydro 2000S (A); Analysis model: general purpose; Particle RI: 1.7; Absorption: 0.01; Dispersant RI: 1.4; Obscuration: 10-30%; Mixing (rpm): 2500; Ultrasound: 30%; Measurement: after 3 min (US switched off);

### Electron microscope images:

For the electron microscope images, the samples were mounted on stubs with double faced adhesive tape and sputter coated with 7 nm platinum layers in a high vacuum sputter coater (Leica EM SCD 500). Surface topography was analyzed with a scanning electron microscope (JSM FE SEM 7001, Jeol, Tokyo, Japan). An acceleration voltage of 1.5 kV and a working distances of 7 - 10 mm were used.

### X-Ray powder diffraction method:

Conditions for obtaining powder X-ray diffraction (XRD) patterns: X-ray powder diffraction patterns (XRPD) were obtained with a PANalytical X'Pert PRO diffractometer equipped with a theta/theta coupled goniometer in transmission geometry, Cu-Kα1,2 radiation (wavelength 0,15419 nm) with a focusing mirror and a solid state PIXcel detector. The patterns were recorded at a tube voltage of 45 kV and a tube current of 40 mA, applying a stepsize of 0.013° 2-Theta with 80s per step (255 channels) in the angular range of 2° to 40° 2-Theta at ambient conditions. A typical precision of the 2-theta values is in the range of about ± 0.2° 2-Theta. Thus a diffraction peak that appears at 5.0° 2-Theta can appear between 4.8 and 5.2° 2-Theta on most X-ray diffractometers under standard conditions.

### Differential Scanning Calorimetry:

Conditions for obtaining DSC thermograms: Thermograms were obtained with Mettler Polymer DSC R instrument. About 1 - 2 mg sample were heated in 40 µl aluminium pans with pierced aluminium lids from 25 to 200 °C at a rate of 10 °C/min. Nitrogen (purge rate 50 mL/min) was used as purge gas.

## Claims

1. Crystalline ticagrelor in form of spherical particles.

2. Spherical ticagrelor particles according to claim 1 wherein ticagrelor is in the crystalline form II, the crystalline form III, or a mixture of crystalline forms II and III.

3. A process for the preparation of crystalline spherical ticagrelor particles according to claim 1, comprising the steps of:
(i) providing a solution of ticagrelor in a solvent,
(ii) adding the obtained solution to an antisolvent,
(iii) isolating the obtained precipitated crystalline spherical ticagrelor particles, and
(iv) drying the isolated crystalline spherical ticagrelor particles,
**characterized in that** the precipitation of the spherical ticagrelor crystals is effected at a temperature not exceeding about 10 °C.

4. The process according to claim 3, wherein the solvent is selected from the group consisting of methyl acetate, ethyl acetate, n-propyl acetate, isopropyl acetate, n-butyl acetate, isobutyl acetate, and a mixture thereof.

5. The process according to claim 3, wherein the antisolvent is selected from the group consisting of toluene, n-heptane, isooctane, and a mixture thereof.

6. A process for the preparation of crystalline spherical ticagrelor particles according to claim 1, comprising the steps of:
(i) providing a solution of ticagrelor in a solvent,
(ii) adding an antisolvent to the obtained solution,
(iii) isolating the obtained precipitated crystalline spherical ticagrelor particles, and
(iv) drying the isolated crystalline spherical ticagrelor particles
**characterized in that** the antisolvent is added to the obtained solution of step (i) within about 60 minutes from the start of addition of the antisolvent into the solution, and **in that** the temperature during step (ii) is kept in the range from about 45 to about 55°C.

7. The process according to claim 6, wherein the antisolvent is added to the obtained solution of step (i) within about 30 minutes from the start of addition of the antisolvent into the solution, preferably within about 15 minutes from the start of addition of the antisolvent into the solution.

8. The process according to claim 6, wherein the solvent is selected from the group consisting of methyl acetate, ethyl acetate, n-propyl acetate, isopropyl acetate, n-butyl acetate, isobutyl acetate, and a mixture thereof.

9. A process according to claim 6, wherein the antisolvent is selected from the group consisting of toluene, n-heptane, isooctane, and a mixture thereof.

10. The process according to claim 6, wherein the temperature during step (ii) is kept at about 50°C and wherein step (iii) is performed within 1 hour.

11. Crystalline ticagrelor in form of spherical particles for use in medicine.

12. Crystalline ticagrelor in form of spherical particles for use in the treatment or prevention of arterial thrombotic complications in patients with coronary artery, cerebrovascular or peripheral vascular disease.

13. A pharmaceutical composition comprising crystalline ticagrelor in form of spherical particles and a pharmaceutically acceptable carrier or diluent.

14. The pharmaceutical composition according to claim 13, wherein said pharmaceutical composition is in a form of a tablet.

15. A process for preparing the pharmaceutical composition according to claim 14 comprising the steps of:
a) providing crystalline ticagrelor in form of spherical particles,
b) dry mixing the provided crystalline ticagrelor in form of spherical particles with the pharmaceutically acceptable carrier or diluent, and
c) directly compressing the obtained admixture into a tablet.
